# EUROPEAN PATENT SPECIFICATION

(11) **EP 4 431 066 B1**
(45) Date of publication and mention of the grant of the patent: **09.04.2025**
(21) Application number: 23212263.0
(22) Date of filing: 27.11.2023
(51) Int. Cl.: A61F 5/451, A61F 5/455, A61M 1/00

(54) **FLEXIBLE WALL STORAGE UNIT FOR URINARY RELIEF SYSTEM**
FLEXIBLE WANDAUFBEWAHRUNGSEINHEIT FÜR HARNENTLASTUNGSSYSTEM
UNITÉ DE STOCKAGE DE PAROI SOUPLE POUR SYSTÈME DE SOULAGEMENT URINAIRE

(30) Priority: 17.03.2023 US 202318186065
(43) Date of publication of application: 18.09.2024
(73) Proprietor: B/E Aerospace, Inc., Winston-Salem, NC 27105 (US)
(72) Inventor: KUCZEK, Andrzej E., Bristol, 06010 (US); KHAKPOUR, Yasmin, South Windsor, 06074 (US); PEARSON, Matthew R., Hartford, 06106 (US)
(74) Representative: Dehns

(56) References cited:
- EP-B1- 1 504 737
- WO-A1-2021/211801
- WO-A1-2021/231532
- US-A1- 2022 133 977
- US-A1- 2022 370 237

## Description

### GOVERNMENT LICENSE RIGHTS

This disclosure was made with government support under contract No. FA8606-22-9-0002 awarded by the United States Air Force. The government has certain rights in the disclosure.

### FIELD

The present disclosure generally relates urinary relief systems, and more specifically, to pressurized urinary relief systems for pilots.

### BACKGROUND

Aircrew often need to urinate multiple times during flights without removing restraint systems and flight equipment. Current mission profiles and air refueling abilities have led to longer flight times for aircrew. As a result, some aircrew, especially female aircrew, resort to ingesting fewer liquids to reduce the need to urinate resulting in dehydration. A safe, reliable, and effective system to provide aircrew, especially female aircrew, the capability of bladder relief during flight is sought. US 2022/133977 relates to a fluid collection device. EP 1 504 737 relates to a urine disposal device and urine receptacle.

### SUMMARY

Disclosed herein is a storage unit for a urinary relief system. The storage unit includes a body, an inlet port coupled to the body, an outlet port coupled to the body, a first porous layer disposed within the body and adjacent the inlet port, an absorbent layer disposed within the body and adjacent the first porous layer, and a second porous layer disposed within the body and adjacent the absorbent layer and the outlet port.

In various embodiments, the body is configured to be collapsible. In various embodiments, the storage unit further includes a flow restrictor configured to prevent liquid from exiting the storage unit. In various embodiments, the flow restrictor is disposed within the body. In various embodiments, the body is made from one or more of a plastic, a rubber, and a vinyl.

The absorbent layer is between the first porous layer and the second porous layer, and wherein the absorbent layer is laterally adjacent the first porous layer and the second porous layer. Alternatively, the absorbent layer is above the first porous layer and the absorbent layer is below the second porous layer.

Also disclosed herein is a urinary relief system including a human interface device, a control unit, and a storage device coupled to the human interface device and the control unit. The storage device includes an inlet port coupled to the human interface device, an outlet port coupled to the control unit, a first porous layer disposed adjacent the inlet port, an absorbent layer disposed adjacent the first porous layer, and a second porous layer disposed adjacent the absorbent layer and the outlet port.

In various embodiments, the storage device is configured to be collapsible. In various embodiments, the storage device further includes a body disposed around the first porous layer, the absorbent layer, and the second porous layer, the body being made from one or more of a plastic, a rubber, and a vinyl. In various embodiments, the urinary relief system further includes a flow restrictor configured to prevent a liquid from reaching the control unit.

In various embodiments, the flow restrictor is disposed between the second porous layer and the outlet port. In various embodiments, the absorbent layer is between the first porous layer and the second porous layer, and wherein the absorbent layer is laterally adjacent the first porous layer and the second porous layer. In various embodiments, the absorbent layer is above the first porous layer and the absorbent layer is below the second porous layer.

Also disclosed herein is a urinary relief system including a human interface device, a control unit, a flow restrictor, and a storage device coupled to the human interface device by a first tube and the control unit by a second tube, where the flow restrictor is between the control unit and the storage device along the second tube. The storage device includes an inlet port coupled to the first tube, an outlet port coupled to the second tube, a first porous layer disposed adjacent the inlet port, an absorbent layer disposed adjacent the first porous layer, and a second porous layer disposed adjacent the absorbent layer and the outlet port.

In various embodiments, the storage device is configured to be collapsible. In various embodiments, the storage device further includes a body disposed around the first porous layer, the absorbent layer, and the second porous layer, the body being made from one or more of a plastic, a rubber, and a vinyl. In various embodiments, the flow restrictor prevents a liquid from reaching the control unit. In various embodiments, the absorbent layer is between the first porous layer and the second porous layer, and wherein the absorbent layer is laterally adjacent the first porous layer and the second porous layer. In various embodiments, the absorbent layer is above the first porous layer and the absorbent layer is below the second porous layer.

The foregoing features and elements may be combined in any combination, without exclusivity, unless expressly indicated herein otherwise. These features and elements as well as the operation of the disclosed embodiments will become more apparent in light of the following description and accompanying drawings.

### BRIEF DESCRIPTION OF THE DRAWINGS

The subject matter of the present disclosure is particularly pointed out and distinctly claimed in the concluding portion of the specification. A more complete understanding of the present disclosure, however, may best be obtained by referring to the following detailed description and claims in connection with the following drawings. While the drawings illustrate various embodiments employing the principles described herein, the drawings do not limit the scope of the claims.
FIGS. 1A and 1B illustrate a urinary relief system, in accordance with various embodiments.
FIG. 2 illustrates a functional diagram of a urinary relief system, in accordance with various embodiments.
FIG. 3 illustrates a storage unit for a urinary relief system, in accordance with various embodiments.
FIG. 4 illustrates a storage unit for a urinary relief system, in accordance with various embodiments.

### DETAILED DESCRIPTION

The following detailed description of various embodiments herein makes reference to the accompanying drawings, which show various embodiments by way of illustration. While these various embodiments are described in sufficient detail to enable those skilled in the art to practice the disclosure, it should be understood that other embodiments may be realized and that changes may be made without departing from the scope of the disclosure. Thus, the detailed description herein is presented for purposes of illustration only and not of limitation. While these exemplary embodiments are described in sufficient detail to enable those skilled in the art to practice the invention, it should be understood that other embodiments may be realized and that logical, chemical and mechanical changes may be made without departing from the scope of the invention. For example, the steps recited in any of the method or process descriptions may be executed in any order and are not necessarily limited to the order presented. Furthermore, any reference to singular includes plural embodiments, and any reference to more than one component or step may include a singular embodiment or step. Also, any reference to attached, fixed, connected, or the like may include permanent, removable, temporary, partial, full or any other possible attachment option. Additionally, any reference to without contact (or similar phrases) may also include reduced contact or minimal contact. It should also be understood that unless specifically stated otherwise, references to "a," an" or "the" may include one or more than one and that reference to an item in the singular may also include the item in the plural. Further, all ranges may include upper and lower values and all ranges and ratio limits disclosed herein may be combined.

Disclosed herein is a urinary relief system including a human interface, a storage unit, and a control unit. The human interface is configured to collect and pass a liquid (e.g., urine) from a user (e.g., a pilot) to the storage unit. In various embodiments, the human interface may be designed to be worn under clothing (e.g., flight suit) and close the body of the user. In various embodiments, the human interface may be fluidly coupled to the storage unit by a hose, or tube, and, in some embodiments, a quick connector coupling. The storage unit is configured to collect and store the liquid (e.g., urine). In various embodiments, the storage unit includes an input fluidly coupled to the human interface device and an output fluidly coupled to the control unit. The control unit is configured to draw the liquid from the human interface to the storage unit for collection. In various embodiments, the control unit may draw the liquid and some air using a vacuum. In various embodiments, the storage unit and the control unit may be coupled by a hose, or tube, and, in various embodiments, a quick connector coupling.

In various embodiments, the storage unit is a collapsible storage unit with the outer portion constructed from plastic, rubber, vinyl, or another pliable material. That is, the storage unit may be smaller when empty than when full. In various embodiments, the storage unit includes a first porous media layer adjacent the input to diffuse, or spread, the liquid and any accompanying air across a larger surface area. In various embodiments, the storage unit includes an absorption layer adjacent the first porous media layer to collect and store the received liquid. In various embodiments, the storage unit includes a second porous media layer adjacent the output to diffuse, or spread, the vacuum pull from the control unit. In various embodiments, the first porous media layer and the second porous media layer improve the storage capability of the storage unit. In various embodiments, the output includes liquid restrictor to prevent liquid from passing to the control unit.

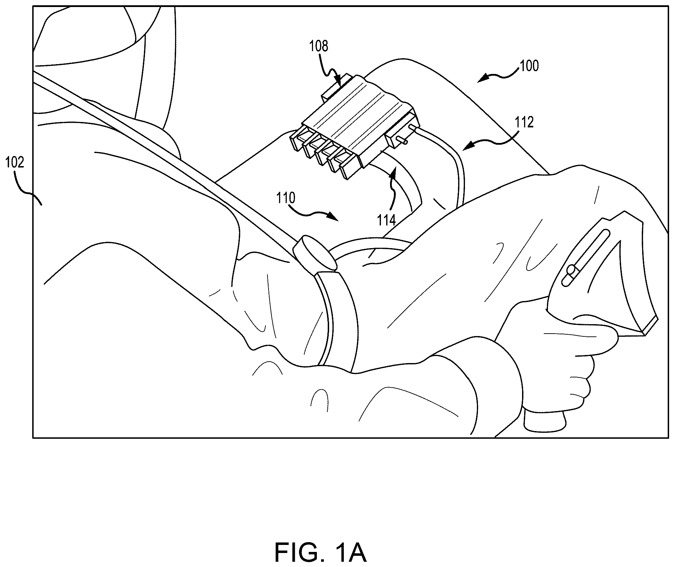

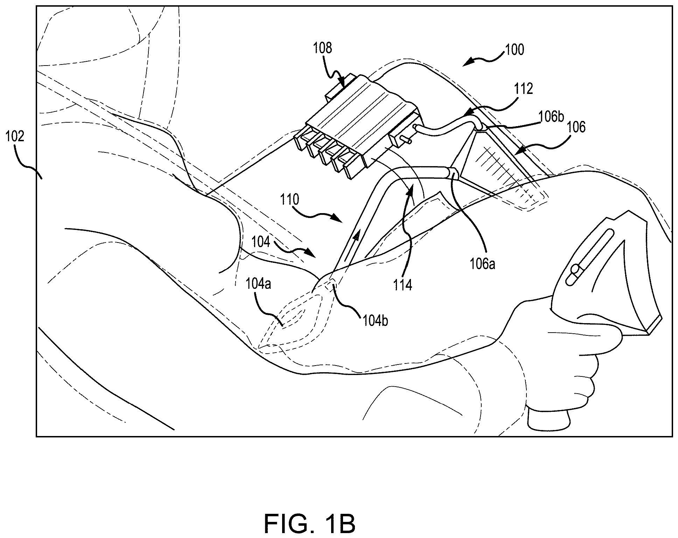

Referring to FIGS. 1A and 1B, a urinary relief system 100 for use by a pilot 102 (e.g., a female pilot) is illustrated, in accordance with various embodiments. Urinary relief system 100 includes a human interface device 104, a storage unit 106, and a control unit 108. Human interface device 104 is configured to be positioned under the clothing (e.g., flight suit, underwear, pants, etc.) and immediately adjacent the body of pilot 102 for use in flight. That is, between the body and clothing of pilot 102 when pilot 102 is in a seated position. In various embodiments, human interface device 104 may form a seal with the body of pilot 102. Human interface device 104 may be any size and/or shape conducive for placement under the clothing of pilot 102 and for collecting the liquid (e.g., urine) while in place. Human interface device 104 includes a collection portion 104a and an outlet port 104b.

Storage unit 106 includes a storage inlet port 106a and a storage outlet port 106b. Outlet port 104b of human interface device 104 is connected to storage inlet port 106a of storage unit 106 by an inlet hose 110 and storage outlet port 106b of storage unit 106 is connected to control unit 108 by an outlet hose 112. In various embodiments, storage unit 106 may be disposable. In various embodiments, control unit 108 and/or storage unit 106 may be secured to pilot 102 (e.g., secured to a leg) by one or more straps 114. Storage unit 106 is configured to receive and trap liquid (e.g., urine) received from storage inlet port 106a and not allow the liquid to exit through storage outlet port 106b.

Control unit 108 creates a motive force that draws the liquid (e.g., urine) from human interface device 104 and into storage unit 106. Air may be drawn, along with the liquid, into storage unit 106 by the vacuum created by control unit 108. In various embodiments, storage unit 106 includes a filter, or flow restrictor, that allows the air to exit storage unit 106 through storage outlet port 106b and keep the liquid secured in storage unit 106. In various embodiments, control unit 108 provides the motive force by creating a vacuum to draw the liquid from human interface device 104 to storage unit 106.

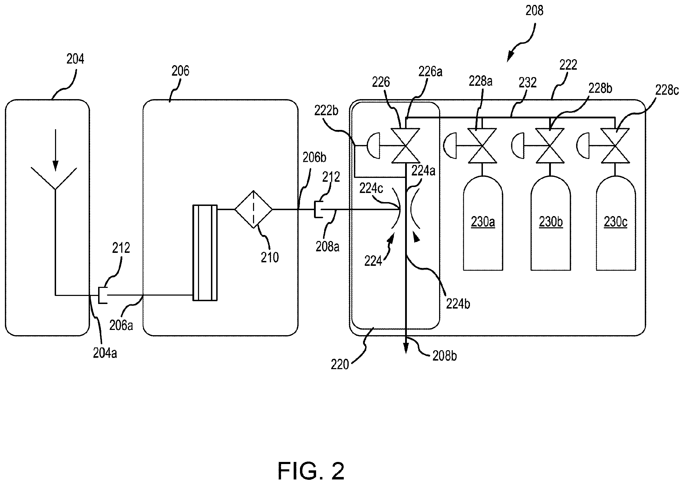

Referring now to FIG. 2, a functional diagram 200 of a urinary relief system is illustrated, in accordance with various embodiments. Functional diagram 200 may be an example of urinary relief system 100 described above with respect to FIGS. 1A and 1B. Functional diagram 200 includes a collection device 204, a storage device 206, and a control device 208. In various embodiments, collection device 204, storage device 206, and control device 208 may be examples of human interface device 104, storage unit 106, and control unit 108, respectively, described above with respect to FIGS. 1A and 1B.

Collection device 204 is configured to collect liquid (e.g., urine) from a user (e.g., pilot 102). Collection device 204 has an outlet port 204a, storage device 206 has a storage inlet port 206a and a storage outlet port 206b, and control device 208 has a control inlet port 208a and a control outlet port 208b. Outlet port 204a of collection device 204 is configured to transfer the collected liquid from collection device 204 to storage device 206. Outlet port 204a of collection device 204 is connected to storage inlet port 206a of storage device 206. Storage outlet port 206b of storage device 206 is connected to control inlet port 208a of control device 208. In various embodiments, a hose, or tube, may be used to make the connection between outlet and inlet ports. In various embodiments, there is a first quick connector interface 212 between collection device 204 and storage device 206 that allows for quick and simple attachment and detachment of a hose, or tube. In various embodiments, there is a second quick connector interface 214 between storage device 206 and control device 208 that allows for quick and simple attachment and detachment the hose, or tube. In various embodiments, a filter, or flow restrictor 210, is connected to storage outlet port 206b of storage device 206. Flow restrictor 210 prevents liquid (e.g., urine) from passing to control device 208 while allowing air to pass to control device 208.

Control device 208 includes an ejector head 220 and a pressurized gas supply 222. Ejector head 220 includes houses control inlet port 208a, control outlet port 208b, an ejector 224, and a pressure regulator 226. Ejector 224 is coupled to pressure regulator 226, control inlet port 208a, and control outlet port 208b. Pressurized gas supply 222 includes a first valve 228a, a second valve 228b, a third valve 228c, a first CO₂ cartridge 230a, a second CO₂ cartridge 230b, and a third CO₂ cartridge 230c. First CO₂ cartridge 230a is coupled to first valve 228a, second CO₂ cartridge 230b is coupled to second valve 228b, and third CO₂ cartridge 230c is coupled to third valve 228c. First valve 228a, second valve 228b, and third valve 228c are connected in parallel to pressure regulator 226 by a high pressure line 232. That is, each valve 228a, 228b, 228c has an independent high pressure connection to high pressure line 232 that is in turn connected to pressure regulator 226. While the description herein uses CO₂ cartridges, it should be appreciated that any suitable pressurized gas container may be used.

Each CO₂ cartridge 230a, 226b, 226c is sealed and connected to the corresponding valve 228a, 228b, 228c. Each valve 228a, 228b, 228c performs the same function. For example, first valve 228a is configured to puncture the seal of first CO₂ cartridge 230a, in response to an input, allowing the compressed air in first CO₂ cartridge 230a to pass through first valve 228a, into high pressure line 232, and to pressure regulator 226. Second valve 228b is configured to puncture the seal of second CO₂ cartridge 230b, in response to an input, allowing the compressed air in second CO₂ cartridge 230b to pass through second valve 228b, into high pressure line 232, and to pressure regulator 226. Third valve 228c is configured to puncture the seal of third CO₂ cartridge 230c, in response to an input, allowing the compressed air in third CO₂ cartridge 230c to pass through third valve 228c, into high pressure line 232, and to pressure regulator 226.

Pressure regulator 226 has a high pressure inlet 226a and a reduced pressure outlet 226b. Pressure regulator 226 receives the high pressure air from high pressure line 232 at high pressure inlet 226a, reduces the pressure of the air (e.g., CO₂), and outputs the air at a reduced pressure from reduced pressure outlet 226b. The pressure of the air at reduced pressure outlet 226b is about 15 times to about 45 times less than the pressure of the air received at high pressure inlet 226a. In various embodiments, the pressure of the air at reduced pressure outlet 226b is about 25 times to about 35 times less than the pressure of the air received at high pressure inlet 226a. Reducing the pressure of the air by pressure regulator 226 slows the release of the pressurized air (e.g., CO₂) and therefore extends the lifespan of each CO₂ cartridge 230a, 266b, 226c. Adjusting the release pressure (e.g., air pressure at reduced pressure outlet 226b) either up or down may decrease or increase, respectively, the effective lifespan of each CO₂ cartridge 230a, 230b, 230c.

Ejector 224 has an air inlet 224a, an air outlet 224b, and a vacuum port 224c. Air inlet 224a of ejector 224 is connected to reduced pressure outlet 226b of pressure regulator 226. Air outlet 224b is connected to control outlet port 208b which is open to the environment (e.g., cockpit, cabin, etc.). Vacuum port 224c is connected to storage outlet port 206b of storage device 206. Ejector 224 receives pressurized air at air inlet 224a and passes the pressurized air to air outlet 224b to create a vacuum at vacuum port 224c. The vacuum creates a motive force that passes through storage device 206 to collection device 204 and draws, or sucks, air and liquid (e.g., urine) from collection device 204 into storage device 206. A combination of the materials inside storage device 206 and flow restrictor 210 trap and prevent the liquid from being pulled to ejector head 220, and more specifically, into ejector 224. Instead, any air present in the system is drawn by the vacuum created by ejector 224, continuing to pull liquid from collection device 204.

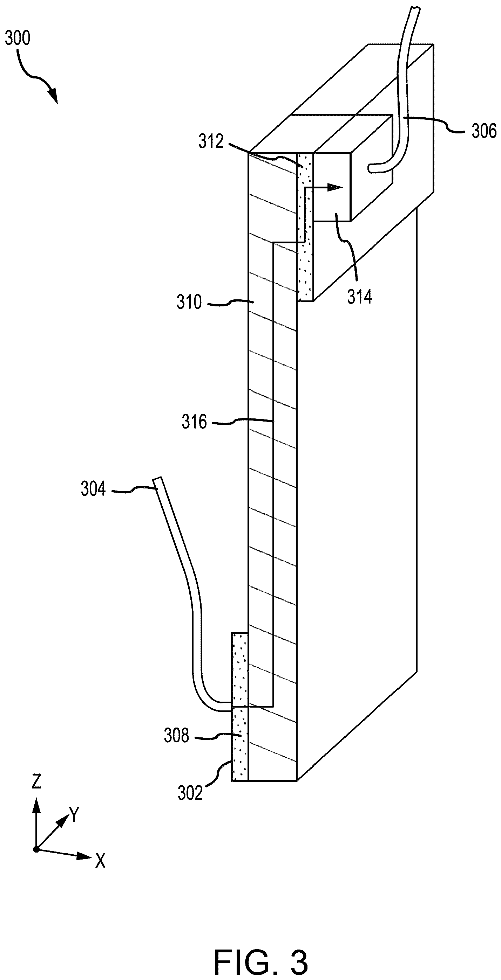

Referring now to FIG. 3, a storage device 300 is illustrated, in accordance with various embodiments. Storage device 300 includes similar components to those described above with respect to storage device 206 in FIG. 2, including an inlet port 304, an outlet port 306, and a flow restrictor 314. Storage device 300 further includes a body 302, a first porous layer 308, an absorbent layer 310, and a second porous layer 312. Storage device 300 as disclosed herein may be disposable thereby simplifying the maintenance of urinary relief system 200.

Body 302 of storage device 300, in various embodiments, may be a multilayer flexible body formed in one of various shapes including be rectangular, triangular, oval, or other shapes to fit given space parameters. In various embodiments, body 302 may be made from a flexible plastic, a flexible rubber, a vinyl material, or other flexible materials. That is, storage device 300 may be designed to be worn by a user (e.g., pilot 102), stored in or on a seat, or stored in or on an aircraft wall, among others. As such, body 302 may be in a collapsed state when empty and in an expanded state when liquid (e.g., urine) is stored therein.

Inlet port 304 may be connected to a collection device (e.g., collection device 204) and outlet port 306 may be connected to a control unit (e.g., control device 208). In the illustrated embodiment, inlet port 304 is located at the bottom of body 302 (e.g., the negative z-direction) and on the left side of body 302 (e.g., the negative x-direction). Outlet port 306 is located the top of body 302 (e.g., the positive z-direction) and on the right side of body 302 (e.g., the positive x-direction). In various embodiments, both inlet port 304 and outlet port 306 may be located at the bottom of body 302 or at the top of body 302. In various embodiments, inlet port 304 may be located at the top of body 302 and the outlet port 306 may be located at the bottom of body 302. Similarly, in various embodiments, inlet port 304 and outlet port 306 may be located on the left side of body 302 or the right side of body 302 or some combination thereof.

First porous layer 308 is located inside body 302 and laterally adjacent inlet port 304. Absorbent layer 310 is located inside body 302 and laterally adjacent first porous layer 308. That is, first porous layer 308 is between inlet port 304 and absorbent layer 310. Second porous layer 312 is located inside body 302 and laterally adjacent absorbent layer 310. That is, absorbent layer 310 is between first porous layer 308 and second porous layer 312. Flow restrictor 314 is located inside body 302 and is laterally adjacent second porous layer 312 on one side and laterally adjacent outlet port 306 on the other side. That is, second porous layer 312 is between flow restrictor 314 and absorbent layer 310 and flow restrictor 314 is between second porous layer 312 and outlet port 306. In various embodiments, flow restrictor 314 may be located external of body 302 so that second porous layer 312 is laterally adjacent outlet port 306.

This configuration provides structure to storage device 300 so that body 302 does not completely collapse when empty which may prevent the control unit (e.g., control device 208) from drawing liquid into storage device 300. When empty, the structure of storage device 300 allows liquid (e.g., urine) and air to enter storage device 300 through inlet port 304 and allows only air to exit storage device 300 through outlet port 306. In the illustrated embodiment, the liquid and/or air has a flow path 316 entering body 302 through inlet port 304 and passing through first porous layer 308 where liquid is absorbed by absorbent layer 310. Flow path 316 as illustrated, indicates that the liquid and air may travel up absorbent layer 310 (e.g., in the positive z-direction) toward outlet port 306. As described above in FIG. 2, a motive force is provided to move the liquid and air into storage device 300, trap the liquid in storage device 300, and allow air to exit storage device 300. In various embodiments, the motive force may be a vacuum (e.g., negative pressure, in the direction towards the outlet port 306) pulling from outlet port 306. In various embodiments, the motive force may be a positive pressure force (e.g., positive pressure coming from the direction of inlet tube 304) that pushes the liquid and air into body 302 through inlet port 304.

First porous layer 308 and second porous layer 312 provide a surface over which to distribute the motive force, the liquid, and/or the air. First porous layer 308 provides a surface over which to distribute the liquid and/or air received from the collection device across the portion of absorbent layer 310 adjacent first porous layer 308. Second porous layer 312 provides a surface over which to distribute the motive force (e.g., vacuum) from the control unit to the portion of absorbent layer 310 adjacent second porous layer 312. In various embodiments, porous layers 308, 312 may be formed from any solid material containing spaces, holes, or voids, either naturally or artificially. In various embodiments, porous layers 308, 312 may be sponges, wood, plastic, pumice, ceramic, or closed-cell extruded polystyrene foam (e.g., that sold under the name STYROFOAM), among others.

Absorbent layer 310 provides a material in which to trap and store the liquid (e.g., urine) received from the collection device (e.g., collection device 204). Absorbent layer 310 may be made from one or more different materials, including polymers, sodium polyacrylate, wood pulp, absorbent beads, or any other suitable absorbent material. In various embodiments, absorbent layer 310 may be formed into a single component prior to being placed inside body 302. In various embodiments, absorbent layer 310 may include multiple layers of different materials that loosely fit together inside body 302. Regardless, of the material used or the form in which it is present, absorbent layer 310 is configured to trap and secure the liquid (e.g., urine) received from the collection device (e.g., collection device 204).

Flow restrictor 314 is located between absorbent layer 310 and the control unit (e.g., control unit 206). Flow restrictor 314 may be a material, a device, or a combination of both that allows air to pass through and prevents the liquid (e.g., urine) from passing. Flow restrictor 314 protects the control unit from getting wet by preventing the liquid from passing. This reduces maintenance for urinary relief system 200. In the illustrated embodiments, the vertical configuration of absorbent layer 310 may aid flow restrictor 314 in preventing the liquid from passing. That is, gravity may act on the liquid decreasing its ability to rise to the top (e.g., the positive z-direction) of absorbent layer 310 as compared to the air.

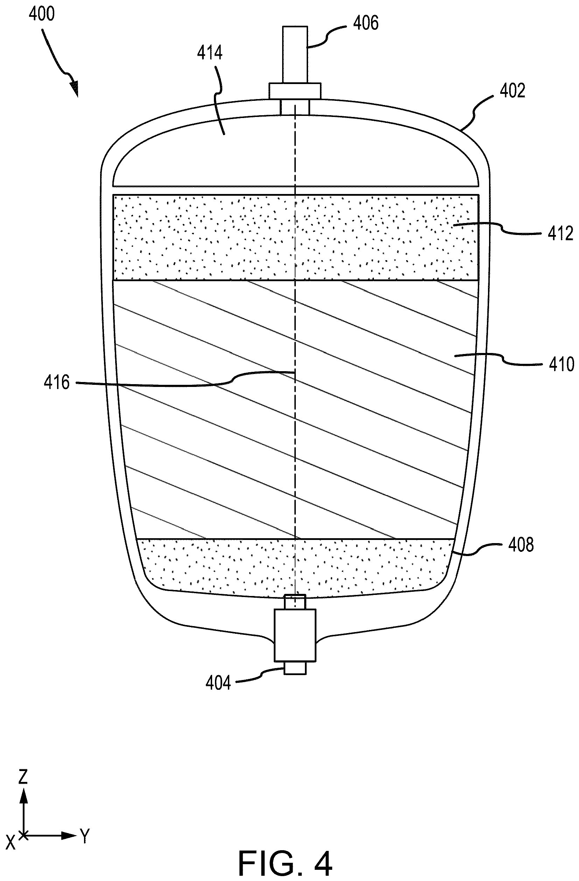

Referring now to FIG. 4, a storage device 400 is illustrated, in accordance with various embodiments. Storage device 400 includes similar components to those described above with respect to storage device 300 in FIG. 3, including a body 402, an inlet port 404, an outlet port 406, a first porous layer 408, an absorbent layer 410, a second porous layer 412, and a flow restrictor 414. Similar to storage device 300, storage device 400 provides a flow path 416 for the liquid and/or air through the various layers of storage device 400.

In the illustrated embodiment, the various layers of storage device 400 are arranged so that they are vertically adjacent (e.g., along the z-axis) instead of horizontally adjacent (e.g., along the x-axis) as described above with respect to storage device 300 in FIG. 3. That is, first porous layer 408 is above (e.g., in the positive z-direction) inlet port 404, absorbent layer 410 is above (e.g., in the positive z-direction) first porous layer 408, second porous layer 412 is above (e.g., in the positive z-direction) the absorbent layer 410, flow restrictor 414 is above (e.g., in the positive z-direction) second porous layer 412, and outlet port 406 is above (e.g., in the positive z-direction) flow restrictor 414. In various embodiments, flow restrictor 414 may be located outside of body 402 and along a tube coupled to outlet port 406.

It should be appreciated that other configurations of storage device 400 may be achieved by varying the size, shape, and layout of body 402 and the various materials disposed therein (i.e., first porous layer 408, absorbent layer 410, second porous layer 412, and flow restrictor 414). Accordingly, the examples described and illustrated herein are not intended to be limiting as to the form factor of storage device 400.

Benefits, other advantages, and solutions to problems have been described herein with regard to specific embodiments. Furthermore, the connecting lines shown in the various figures contained herein are intended to represent exemplary functional relationships and/or physical couplings between the various elements. It should be noted that many alternative or additional functional relationships or physical connections may be present in a practical system. However, the benefits, advantages, solutions to problems, and any elements that may cause any benefit, advantage, or solution to occur or become more pronounced are not to be construed as critical, required, or essential features or elements of the disclosure. The scope of the disclosure is accordingly to be limited by nothing other than the appended claims, in which reference to an element in the singular is not intended to mean "one and only one" unless explicitly so stated, but rather "one or more." Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C. Different cross-hatching is used throughout the figures to denote different parts but not necessarily to denote the same or different materials.

Systems, methods and apparatus are provided herein. In the detailed description herein, references to "one embodiment," "an embodiment," "various embodiments," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

Numbers, percentages, or other values stated herein are intended to include that value, and also other values that are about or approximately equal to the stated value, as would be appreciated by one of ordinary skill in the art encompassed by various embodiments of the present disclosure. A stated value should therefore be interpreted broadly enough to encompass values that are at least close enough to the stated value to perform a desired function or achieve a desired result. The stated values include at least the variation to be expected in a suitable industrial process, and may include values that are within 5% of a stated value. Additionally, the terms "substantially," "about" or "approximately" as used herein represent an amount close to the stated amount that still performs a desired function or achieves a desired result. For example, the term "substantially," "about" or "approximately" may refer to an amount that is within 5% of a stated amount or value.

Furthermore, no element, component, or method step in the present disclosure is intended to be dedicated to the public regardless of whether the element, component, or method step is explicitly recited in the claims. As used herein, the terms "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus.

Finally, it should be understood that any of the above-described concepts can be used alone or in combination with any or all of the other above-described concepts. Although various embodiments have been disclosed and described, one of ordinary skill in this art would recognize that certain modifications would come within the scope of this disclosure. Accordingly, the description is not intended to be exhaustive or to limit the principles described or illustrated herein to any precise form. Many modifications and variations are possible in light of the above teaching.

## Claims

1. A storage unit for a urinary relief system, comprising:
a body (302);
an inlet port (304) coupled to the body;
an outlet port (306) coupled to the body;
a first porous layer (308) disposed within the body and adjacent the inlet port (304);
an absorbent layer (310) disposed within the body and adjacent the first porous layer; and
a second porous layer (312) disposed within the body and adjacent the absorbent layer and the outlet port (306);
**characterized in that**
the absorbent layer (310) is between the first porous layer (308) and the second porous layer (312), and wherein the absorbent layer (310) is laterally adjacent the first porous layer (308) and the second porous layer (312); or
wherein the absorbent layer (310) is above the first porous layer (308) and the absorbent layer is below the second porous layer (312).

2. The storage unit of claim 1, wherein the body (302) is configured to be collapsible.

3. The storage unit of claim 1 or 2, further comprising:
a flow restrictor (314) configured to prevent liquid from exiting the storage unit.

4. The storage unit of claim 3, wherein the flow restrictor (314) is disposed within the body (302).

5. The storage unit of any preceding claim, wherein the body (302) is made from one or more of a plastic, a rubber, and a vinyl.

6. A urinary relief system, comprising:
a human interface device (104);
a control unit (108); and
a storage device coupled to the human interface device (104) and the control unit (108);
**characterized in that** the storage device includes :
an inlet port (304) coupled to the human interface device (104);
an outlet port (306) coupled to the control unit (108);
a first porous layer (308) disposed adjacent the inlet port (304);
an absorbent layer (310) disposed adjacent the first porous layer (308); and
a second porous layer (312) disposed adjacent the absorbent layer (310) and the outlet port (306);
wherein the absorbent layer (310) is between the first porous layer (308) and the second porous layer (312), and wherein the absorbent layer (310) is laterally adjacent the first porous layer (308) and the second porous layer (312); or
wherein the absorbent layer (310) is above the first porous layer (308) and the absorbent layer is below the second porous layer (312).

7. The urinary relief system of claim 6, wherein the storage device is configured to be collapsible.

8. The urinary relief system of claim 7, wherein the storage device further comprises:
a body (302) disposed around the first porous layer (308), the absorbent layer (310), and the second porous layer (312), the body (302) being made from one or more of a plastic, a rubber, and a vinyl.

9. The urinary relief system of claim 6, 7, or 8, further comprising:
a flow restrictor (314) configured to prevent a liquid from reaching the control unit (108).

10. The urinary relief system of claim 9, wherein the flow restrictor (314) is disposed between the second porous layer (312) and the outlet port (306).

11. The urinary relief system of claim 6, further comprising:
a flow restrictor (314); and
wherein the storage device is coupled to the human interface device (104) by a first tube and the control unit (108) by a second tube, wherein the flow restrictor (314) is between the control unit (108) and the storage device along the second tube, wherein:
the inlet port (304) is coupled to the first tube; and
the outlet port (306) is coupled to the second tube.

12. The urinary relief system of claim 11, wherein the storage device is configured to be collapsible.

13. The urinary relief system of claim 12, wherein the storage device further comprises:
a body (302) disposed around the first porous layer (308), the absorbent layer (310), and the second porous layer (312), the body (302) being made from one or more of a plastic, a rubber, and a vinyl.

14. The urinary relief system of claim 11, 12, or 13, wherein the flow restrictor (314) prevents a liquid from reaching the control unit (108).

## Patentansprüche

1. Aufbewahrungseinheit für ein Harnentlastungssystem, umfassend:
einen Körper (302);
einen Einlassanschluss (304), der mit dem Körper gekoppelt ist; einen Auslassanschluss (306), der mit dem Körper gekoppelt ist; eine erste poröse Schicht (308), die innerhalb des Körpers und benachbart an dem Einlassanschluss (304) angeordnet ist;
eine absorbierende Schicht (310), die innerhalb des Körpers und benachbart an der ersten porösen Schicht angeordnet ist; und
eine zweite poröse Schicht (312), die innerhalb des Körpers und benachbart an der absorbierenden Schicht und dem Auslassanschluss (306) angeordnet ist;
**dadurch gekennzeichnet, dass**
die absorbierende Schicht (310) zwischen der ersten porösen Schicht (308) und der zweiten porösen Schicht (312) liegt, und wobei die absorbierende Schicht (310) seitlich benachbart an der ersten porösen Schicht (308) und der zweiten porösen Schicht (312) liegt; oder
wobei die absorbierende Schicht (310) über der ersten porösen Schicht (308) liegt und die absorbierende Schicht unter der zweiten porösen Schicht (312) liegt.

2. Aufbewahrungseinheit nach Anspruch 1, wobei der Körper (302) dazu konfiguriert ist, zusammenklappbar zu sein.

3. Aufbewahrungseinheit nach Anspruch 1 oder 2, ferner umfassend:
einen Durchflussbegrenzer (314), der dazu konfiguriert ist, ein Austreten von Flüssigkeit aus der Aufbewahrungseinheit zu verhindern.

4. Aufbewahrungseinheit nach Anspruch 3, wobei der Durchflussbegrenzer (314) innerhalb des Körpers (302) angeordnet ist.

5. Aufbewahrungseinheit nach einem der vorhergehenden Ansprüche, wobei der Körper (302) aus einem oder mehreren von einem Kunststoff, einem Gummi und einem Vinyl hergestellt ist.

6. Harnentlastungssystem, umfassend:
ein Mensch-Schnittstellenvorrichtung (104);
eine Steuereinheit (108); und
eine Aufbewahrungsvorrichtung, die mit der Mensch-Schnittstellenvorrichtung (104) und der Steuereinheit (108) gekoppelt ist;
**dadurch gekennzeichnet, dass** die Aufbewahrungsvorrichtung Folgendes beinhaltet:
einen Einlassanschluss (304), der mit der Mensch-Schnittstellenvorrichtung (104) gekoppelt ist;
einen Auslassanschluss (306), der mit der Steuereinheit (108) gekoppelt ist;
eine erste poröse Schicht (308), die benachbart an dem Einlassanschluss (304) angeordnet ist;
eine absorbierende Schicht (310), die benachbart an der ersten porösen Schicht (308) angeordnet ist; und
eine zweite poröse Schicht (312), die benachbart an der absorbierenden Schicht (310) und dem Auslassanschluss (306) angeordnet ist;
wobei die absorbierende Schicht (310) zwischen der ersten porösen Schicht (308) und der zweiten porösen Schicht (312) liegt, und wobei die absorbierende Schicht (310) seitlich benachbart an der ersten porösen Schicht (308) und der zweiten porösen Schicht (312) liegt; oder
wobei die absorbierende Schicht (310) über der ersten porösen Schicht (308) liegt und die absorbierende Schicht unter der zweiten porösen Schicht (312) liegt.

7. Harnentlastungssystem nach Anspruch 6, wobei die Aufbewahrungsvorrichtung dazu konfiguriert ist, zusammenklappbar zu sein.

8. Harnentlastungssystem nach Anspruch 7, wobei die Aufbewahrungsvorrichtung ferner Folgendes umfasst:
einen Körper (302), der um die erste poröse Schicht (308), die absorbierende Schicht (310) und die zweite poröse Schicht (312) angeordnet ist, wobei der Körper (302) aus einem oder mehreren von einem Kunststoff, einem Gummi und einem Vinyl hergestellt ist.

9. Harnentlastungssystem nach Anspruch 6, 7 oder 8, ferner umfassend:
einen Durchflussbegrenzer (314), der dazu konfiguriert ist, zu verhindern, dass eine Flüssigkeit die Steuereinheit (108) erreicht.

10. Harnentlastungssystem nach Anspruch 9, wobei der Durchflussbegrenzer (314) zwischen der zweiten porösen Schicht (312) und dem Auslassanschluss (306) angeordnet ist.

11. Harnentlastungssystem nach Anspruch 6, ferner umfassend:
einen Durchflussbegrenzer (314); und
wobei die Aufbewahrungsvorrichtung durch ein erstes Rohr mit der Mensch-Schnittstellenvorrichtung (104) und durch ein zweites Rohr mit der Steuereinheit (108) gekoppelt ist, wobei der Durchflussbegrenzer (314) zwischen der Steuereinheit (108) und der Aufbewahrungsvorrichtung entlang des zweiten Rohrs liegt,
wobei:
der Einlassanschluss (304) mit dem ersten Rohr gekoppelt ist; und
der Auslassanschluss (306) mit dem zweiten Rohr gekoppelt ist.

12. Harnentlastungssystem nach Anspruch 11, wobei die Aufbewahrungsvorrichtung dazu konfiguriert ist, zusammenklappbar zu sein.

13. Harnentlastungssystem nach Anspruch 12, wobei die Aufbewahrungsvorrichtung ferner Folgendes umfasst: einen Körper (302), der um die erste poröse Schicht (308), die absorbierende Schicht (310) und die zweite poröse Schicht (312) angeordnet ist, wobei der Körper (302) aus einem oder mehreren von einem Kunststoff, einem Gummi und einem Vinyl hergestellt ist.

14. Harnentlastungssystem nach Anspruch 11, 12 oder 13, wobei der Durchflussbegrenzer (314) verhindert, dass eine Flüssigkeit die Steuereinheit (108) erreicht.

## Revendications

1. Unité de stockage pour un système de soulagement urinaire, comprenant :
un corps (302) ;
un orifice d'entrée (304) couplé au corps ;
un orifice de sortie (306) couplé au corps ;
une première couche poreuse (308) disposée à l'intérieur du corps et adjacente à l'orifice d'entrée (304) ;
une couche absorbante (310) disposée à l'intérieur du corps et adjacente à la première couche poreuse ; et
une seconde couche poreuse (312) disposée à l'intérieur du corps et adjacente à la couche absorbante et à l'orifice de sortie (306) ;
**caractérisée en ce que**
la couche absorbante (310) est située entre la première couche poreuse (308) et la seconde couche poreuse (312), et dans laquelle la couche absorbante (310) est latéralement adjacente à la première couche poreuse (308) et à la seconde couche poreuse (312) ; ou
dans laquelle la couche absorbante (310) est au-dessus de la première couche poreuse (308) et la couche absorbante est en dessous de la seconde couche poreuse (312).

2. Unité de stockage selon la revendication 1, dans laquelle le corps (302) est configuré pour être repliable.

3. Unité de stockage selon la revendication 1 ou 2, comprenant également :
un limiteur de débit (314) configuré pour empêcher le liquide de sortir de l'unité de stockage.

4. Unité de stockage selon la revendication 3, dans laquelle le limiteur de débit (314) est disposé à l'intérieur du corps (302).

5. Unité de stockage selon une quelconque revendication précédente, dans laquelle le corps (302) est constitué de l'un ou plusieurs d'un plastique, d'un caoutchouc et d'un vinyle.

6. Système de soulagement urinaire, comprenant :
un dispositif d'interface humaine (104) ;
une unité de commande (108) ; et
un dispositif de stockage couplé au dispositif d'interface humaine (104) et à l'unité de commande (108) ;
**caractérisé en ce que** le dispositif de stockage comprend :
un orifice d'entrée (304) couplé au dispositif d'interface humaine (104) ;
un orifice de sortie (306) couplé à l'unité de commande (108) ;
une première couche poreuse (308) disposée adjacente à l'orifice d'entrée (304) ;
une couche absorbante (310) disposée adjacente à la première couche poreuse (308) ; et
une seconde couche poreuse (312) disposée adjacente à la couche absorbante (310) et à l'orifice de sortie (306) ;
dans lequel la couche absorbante (310) est située entre la première couche poreuse (308) et la seconde couche poreuse (312), et dans lequel la couche absorbante (310) est latéralement adjacente à la première couche poreuse (308) et à la seconde couche poreuse (312) ; ou
dans lequel la couche absorbante (310) est au-dessus de la première couche poreuse (308) et la couche absorbante est en dessous de la seconde couche poreuse (312).

7. Système de soulagement urinaire selon la revendication 6, dans lequel le dispositif de stockage est configuré pour être repliable.

8. Système de soulagement urinaire selon la revendication 7, dans lequel le dispositif de stockage comprend également :
un corps (302) disposé autour de la première couche poreuse (308), de la couche absorbante (310) et de la seconde couche poreuse (312), le corps (302) étant constitué de l'un ou plusieurs d'un plastique, d'un caoutchouc et d'un vinyle.

9. Système de soulagement urinaire selon la revendication 6, 7 ou 8, comprenant également :
un limiteur de débit (314) configuré pour empêcher un liquide d'atteindre l'unité de commande (108).

10. Système de soulagement urinaire selon la revendication 9, dans lequel le limiteur de débit (314) est disposé entre la seconde couche poreuse (312) et l'orifice de sortie (306).

11. Système de soulagement urinaire selon la revendication 6, comprenant également :
un limiteur de débit (314) ; et
dans lequel le dispositif de stockage est couplé au dispositif d'interface humaine (104) par un premier tube et à l'unité de commande (108) par un second tube, dans lequel le limiteur de débit (314) est situé entre l'unité de commande (108) et le dispositif de stockage le long du second tube, dans lequel :
l'orifice d'entrée (304) est couplé au premier tube ; et
l'orifice de sortie (306) est couplé au second tube.

12. Système de soulagement urinaire selon la revendication 11, dans lequel le dispositif de stockage est configuré pour être repliable.

13. Système de soulagement urinaire selon la revendication 12, dans lequel le dispositif de stockage comprend également : un corps (302) disposé autour de la première couche poreuse (308), de la couche absorbante (310) et de la seconde couche poreuse (312), le corps (302) étant constitué de l'un ou plusieurs d'un plastique, d'un caoutchouc et d'un vinyle.

14. Système de soulagement urinaire selon la revendication 11, 12 ou 13, dans lequel le limiteur de débit (314) empêche un liquide d'atteindre l'unité de commande (108).
